# EUROPEAN PATENT APPLICATION

(11) **EP 2 870 883 A1**
(43) Date of publication of application: **13.05.2015**
(21) Application number: 13813323.6
(22) Date of filing: 01.07.2013
(51) Int. Cl.: A23K 1/16, A23K 1/18, A61K 9/42, A61K 31/198, A61K 45/00, A61K 47/12, A61K 47/42, A61P 1/00

(54) **COMPOSITION CONTAINING BIOLOGICALLY ACTIVE SUBSTANCE**

(30) Priority: 05.07.2012 JP 2012151349
(71) Applicant: Nisso Shoji Co.,Ltd., Chuo-ku Tokyo 1038422 (JP); NISSO FINE CO., LTD., Chuo-ku Tokyo 103-8422 (JP)
(72) Inventor: MORISHIMA, Ko, Tokyo 100-8165 (JP); ABE, Shuichi, Kitaibaraki-shi Ibaraki 319-1541 (JP); TAKESHI, Yutaka, Kitaibaraki-shi Ibaraki 319-1541 (JP)
(74) Representative: Carlisle, Julie
(86) International application number: PCT/JP2013/004062
(87) International publication number: WO 2014/006867

(57) **Abstract**

An object of the present invention is to provide a composition containing a biologically active substance, which has such an excellent moldability that it can give a favorable granular formulation even when it is not cut with a water-cooled cutter, and which can fulfill the performance required of a rumen-bypassing formulation and the like. The composition containing a biologically active substance of the present invention comprises 10 to 50% by mass of a protective substance comprising a linear or branched, saturated or unsaturated aliphatic C₈₋₂₄ monocarboxylic acid (1a) having a melting point of lower than 50°C, a linear or branched, saturated or unsaturated aliphatic C₈₋₂₄ monocarboxylic acid having a melting point of 50°C or higher, and a calcium salt of a linear or branched, saturated or unsaturated aliphatic C₁₂₋₂₄ monocarboxylic acid (2) having a calcium content of 8 to 12% by mass, and 50 to 90% by mass of a biologically active substance dispersed in the protective substance.

## Description

### Technical field

The present invention relates to a composition containing a biologically active substance. In more detail, the present invention relates to a composition containing a biologically active substance suitable as a rumen-bypassing formulation used for ruminants. The present application claims priority to Japanese Patent Application No. 2012-151349 filed on July 5th, 2012, the contents of which are incorporated herein.

### Background Art

Rumen-bypassing formulations for ruminants are formulations containing various amino acids and vitamins as well as one or more other biologically active substances. The function of rumen-bypassing formulations used for ruminants is to limit the elution and microbial degradation of biologically active substances in the rumen, while enabling the elution and absorption of biologically active substances in digestive organs below the abomasum.

In breeding of ruminants, it has been becoming popular to administer rumen-bypassing formulations with feed for nutritional and clinical benefit. It is economically advantageous in a practical sense and therefore desirable that rumen-bypassing formulations contain high concentrations of biologically active substances. Further, the hardness of formulation is also important in terms of resistance to feed mixing and mastication, and so on.

In relation to the rumen-bypassing formulations mentioned above, patent document 1 discloses a composition containing 10 to 50% by mass of a protective substance, which contains at least one selected from the group consisting of a linear or branched, saturated or unsaturated aliphatic C₈₋₂₄ monocarboxylic acid, a linear or branched, saturated or unsaturated aliphatic C₈₋₂₄ alcohol containing one hydroxyl group, and a linear or branched, saturated or unsaturated aliphatic C₂₋₈ di- or tri-carboxylic acid, and a linear or branched, saturated or unsaturated aliphatic C₁₂₋₂₄ monocarboxylate at a mass ratio of 30:70 to 10:90, and 50 to 90% by mass of a biologically active substance dispersed in the protective substance. Further, this document also discloses a rumen-bypassing formulation for ruminants containing a core formulation consisting of the above composition and a coating layer coating the coreformulation, wherein the coating layer consists of at least one selected from the group consisting of a linear or branched, saturated or unsaturated aliphatic C₁₂₋₂₄ monocarboxylate, a polymer insoluble in the neutral region but soluble in the acidic region, and zein. This document specifically describes, as the protective substance, a substance consisting of a combination of palmitic acid and a calcium salt of a beef tallow fatty acid (calcium concentration: 8.3% by mass), a substance consisting of a combination of lauric acid and a calcium salt of a palm oil fatty acid (calcium concentration: 7.4% by mass), and a substance consisting of a combination of myristic acid and a calcium salt of a palm oil fatty acid (calcium concentration: 7.9% by mass).

### Prior Art Document

### Patent Document

Patent Document 1: WO98/24329

### Summary of the Invention

### Object to be Solved by the Invention

The rumen-bypassing formulation for ruminants described in the aforementioned prior art document is manufactured through complex operations involving kneading and extruding, cutting with a water-cooled cutter, centrifugal dehydration treatment, and then drying.

An object of the present invention is to provide a composition containing a biologically active substance, which has such an excellent moldability that it can give a favorable granular formulation even when it is not cut with a water-cooled cutter, and which can fulfill the performance required of a rumen-bypassing formulation and the like.

### Means to Solve the Object

The present inventors conducted intensive studies to achieve the above-mentioned object. As a result, they have found that a composition containing a biologically active substance, which has such an excellent moldability that it can give a favorable granular formulation even when it is not cut with a water-cooled cutter, and which can fulfill the performance required of a rumen-bypassing formulation and the like can be obtained by using a fatty acid salt containing a specific amount of calcium as a protective substance. The present invention was completed based on the above finding.

That is, the present invention encompasses the following aspects.
[1] A composition comprising
   10 to 50% by mass of a protective substance comprising a linear or branched, saturated or unsaturated aliphatic C₈₋₂₄ monocarboxylic acid (1a) having a melting point of lower than 50°C, a linear or branched, saturated or unsaturated aliphatic C₈₋₂₄ monocarboxylic acid (1b) having a melting point of 50°C or higher, and a calcium salt of a linear or branched, saturated or unsaturated aliphatic C₁₂₋₂₄ monocarboxylic acid (2) having a calcium content of 8 to 12% by mass, and
   50 to 90% by mass of a biologically active substance dispersed in the protective substance.
[2] The composition according to [1], wherein a ratio of a total mass of the aliphatic monocarboxylic acid (1a) and the aliphatic monocarboxylic acid (1b) with respect to a mass of the calcium salt of an aliphatic monocarboxylic acid (2) is 40/60 to 10/90.
[3] The composition according to [1] or [2], wherein a ratio of a mass of the aliphatic monocarboxylic acid (1a) with respect to a mass of the aliphatic monocarboxylic acid (1b) is 80/20 to 20/80.
[4] The composition according to any one of [1] to [3], wherein the biologically active substance is an amino acid.
[5] The composition according to any one of [1] to [3], wherein the biologically active substance is at least one selected from the group consisting of methionine and lysine hydrochloride.
[6] The composition according to any one of [1] to [5], wherein the aliphatic monocarboxylic acid (1a) is lauric acid.
[7] The composition according to any one of [1] to [6], wherein the aliphatic monocarboxylic acid (1b) is at least one selected from the group consisting of palmitic acid, myristic acid, and stearic acid.
[8] The composition according to any one of [1] to [7], wherein the calcium salt of an aliphatic monocarboxylic acid (2) is a calcium salt of a palm oil fatty acid.
[9] A method for producing a composition according to any one of [1] to [8], comprising the steps of:
   kneading and extruding, by an extrusion kneading machine,
      50 to 90% by mass of a biologically active substance and
      10 to 50% by mass of a protective substance comprising a linear or branched, saturated or unsaturated aliphatic C₈₋₂₄ monocarboxylic acid having a melting point of lower than 50°C, a linear or branched, saturated or unsaturated aliphatic C₈₋₂₄ monocarboxylic acid having a melting point of 50°C or higher, and a calcium salt of a linear or branched, saturated or unsaturated aliphatic C₁₂₋₂₄ monocarboxylic acid having a calcium content of 8 to 12% by mass; and
   cutting a resulting product with an air-cooled cutter.
[10] A formulation consisting of a composition according to any one of [1] to [8].
[11] A coated formulation comprising a core formulation consisting of a composition according to any one of [1] to [8] and a coating layer coating the core formulation, wherein the coating layer consists of at least one selected from the group consisting of a linear or branched, saturated or unsaturated aliphatic C₁₂₋₂₄ monocarboxylate, a polymer insoluble in a neutral region but soluble in an acidic region, and zein.
[12] The coated formulation according to [11], wherein an amount of the coating layer is 2 to 20 parts by mass based on 100 parts by mass of the core formulation.
[13] The formulation according to any one of [10] to [12], wherein the formulation is for rumen bypass.

### Effect of the Invention

The composition containing a biologically active substance of the present invention has such an excellent moldability that it can give a favorable granular formulation even when it is not cut with a water-cooled cutter. As a result, the composition containing a biologically active substance of the present invention can be granulated into a formulation using an air-cooled cutter after kneading and extruding; therefore, steps such as dehydration and drying are not necessarily required. Further, the formulation of the present invention can fulfill the performance required of a rumen-bypassing formulation and the like, meaning specifically that, for example, it contains such a high concentration as 50% by mass or more of a biologically active substance and has a low elution rate in the rumen, and so on.

### Mode of Carrying Out the Invention

The composition of the present invention contains a protective substance and a biologically active substance dispersed in the protective substance. The state of dispersion can be confirmed by observing the cross-section of a granule by a scanning electron microscope (SEM). When the biologically active substance is not dispersed in the protective substance, the elution rate of the biologically active substance in the rumen increases, resulting in reduced rumen-bypassing efficiency.

The biologically active substance used in the present invention is a substance which exerts its biological activity upon administration to a ruminant, but is unlikely to be effectively digested and absorbed because of being degraded in the rumen when orally administered. Examples of such a substance include an amino acid such as methionine, lysine or a hydrochloric acid salt thereof, and threonine; an amino acid derivative such as 2-hydroxy-4-methylmercaptobutyric acid and a salt thereof; vitamins such as nicotinic acid, nicotinamide, vitamin A, and vitamin E; sugars such as glucose and fructose; and various veterinary drugs such as an antibiotic and an anthelmintic drug. These biologically active substances are used singly or in combinations of two or more.

The content of the biologically active substance in the composition is normally 50 to 90% by mass, preferably 60 to 85% by mass, more preferably 65 to 80% by mass. It is economically disadvantageous if the content of the biologically active substance is too little. When the content of the biologically active substance is too much, the rumen-bypassing efficiency decreases and production becomes difficult.

The biologically active substance is preferably granular. The granular biologically active substance preferably has a particle diameter of 1 mm or less. Also, the granular biologically active substance preferably contains 50% or more of particles having a size equal to or larger than the opening size of a 250-mesh sieve.

The protective substance used in the present invention contains a calcium salt of a linear or branched, saturated or unsaturated aliphatic C₁₂₋₂₄ monocarboxylic acid, a linear or branched, saturated or unsaturated aliphatic C₈₋₂₄ monocarboxylic acid (1a) having a melting point of lower than 50°C, and a linear or branched, saturated or unsaturated aliphatic C₈₋₂₄ monocarboxylic acid (1b) having a melting point of 50°C or higher.

The content of the protective substance in the composition can be appropriately set depending on the amounts of the biologically active substance and a modifying agent, which is added as needed. The content of the protective substance in the composition is normally 10 to 50% by mass, preferably 15 to 40% by mass, more preferably 20 to 35% by mass.

The calcium salt of a linear or branched, saturated or unsaturated aliphatic monocarboxylic acid used in the present invention is also referred to as bypass fat, which has such a property that it is not degraded in the rumen but digested below the abomasum.

The content of the calcium salt of an aliphatic monocarboxylic acid is preferably 60 to 90% by mass of the protective substance. When the content is out of the above range, it tends to be difficult to obtain a formulation containing a high concentration of biologically active substances with excellent rumen-bypassing efficiency.

The number of carbon atoms in the calcium salt of a linear or branched, saturated or unsaturated aliphatic monocarboxylic acid used in the present invention is 12 to 24, preferably 12 to 18.

When the number of carbon atoms is less than 12, the rumen-bypassing efficiency decreases. When the number of carbon atoms is greater than 24, the digestibility below the abomasum decreases.

Specific examples of the calcium salt of an aliphatic monocarboxylic acid include a calcium salt of lauric acid, palmitic acid, myristic acid, stearic acid, oleic acid, linoleic acid, and linolenic acid. These can be used singly or in combinations of two or more. Among them, particularly in view of the accessibility and cost, a calcium salt of a palm oil fatty acid, a palm kernel oil fatty acid or a coconut oil fatty acid, all of which contain palmitic acid, oleic acid and linoleic acid as the main component, or a fatty acid prepared using, as the raw material, a residue resulting from the removal of a specific fatty acid from any of the above fatty acids by distillation is preferable.

The content of calcium in the calcium salt of a linear or branched, saturated or unsaturated aliphatic monocarboxylic acid used in the present invention is 8 to 12% by mass, preferably 8 to 10% by mass, more preferably 8.5 to 10% by mass. When the Ca concentration is too high, the melting point of a kneaded product increases and moldability into granule by an air-cooled cutter improves, whereas the elution rate in the rumen increases and the rumen-bypassing efficiency decreases. By keeping the calcium content within the above range, the elution rate in the rumen can be reduced, while securing moldability. The calcium content can be measured by a known analytical method. For example, the calcium content can be determined as follows: a calcium salt of a fatty acid is incinerated, and the resulting ash is quantitatively analyzed to calculate the calcium content.

The calcium salt of an aliphatic monocarboxylic acid used as a protective substance preferably has a purity of 90% or more. The purity of the calcium salt of a fatty acid as used herein refers to an insoluble residue (% by mass) remaining after an extraction treatment of a calcium salt of a fatty acid with a solvent such as ethers and ketones. The calculation of insoluble residue excludes adsorbed water. Also, the solvent used in the above extraction treatment is selected from solvents in which contaminating oil and fat dissolve but a calcium salt of a fatty acid does not dissolve.

An aliphatic monocarboxylic acid such as a palm oil fatty acid, which serves as the raw material of the calcium salt of an aliphatic monocarboxylic acid, normally contains approximately 5 to 40% of various additives such as a reaction controller and a stabilizer, for example, triglyceride. A calcium salt of an aliphatic monocarboxylic acid obtained using such an aliphatic monocarboxylic acid as mentioned above is contaminated with impurities such as the aforementioned substances. There is even a commercially available calcium salt of an aliphatic monocarboxylic acid which contains approximately 20% of impurities. Such impurities reduce not only the rumen-bypassing efficiency, but also the hardness of a formulation. Thus, it is preferable that the purity of the calcium salt of an aliphatic monocarboxylic acid be high.

The function of the aliphatic monocarboxylic acids (1a) and (1b), which are used with a calcium salt of an aliphatic monocarboxylic acid as the protective substance of the present invention, is considered to reduce the water-solubility of the protective substance and reduce the crystallinity of the calcium salt of an aliphatic monocarboxylic acid, so as to improve the affinity between the biologically active substance and the protective substance (matrix) to thereby reduce the elution rate of the biologically active substance in the rumen. The amounts of the aliphatic monocarboxylic acids (1a) and (1b) used are preferably in the range within which they are compatible with the calcium salt of an aliphatic monocarboxylic acid. Specifically, the aliphatic monocarboxylic acids (1a) and (1b) are contained in the protective substance in an amount of preferably 10 to 40% by mass, more preferably 20 to 35% by mass. It tends to be difficult to obtain favorable rumen-bypassing efficiency outside the above range.

The number of carbon atoms in the aliphatic monocarboxylic acids (1a) and (1b) used in the present invention is 8 to 24, preferably 12 to 18. When the number of carbon atoms is less than 8, the formulation becomes soft, resulting in reduced rumen-bypassing efficiency. When the number of carbon atoms is greater than 24, the digestibility below the abomasum decreases.

The aliphatic monocarboxylic acid (1a) is the one having a melting point of lower than 50°C. The aliphatic monocarboxylic acid (1b) is the one having a melting point of 50°C or higher.

Although the mass ratio between the aliphatic monocarboxylic acid (1a) and the aliphatic monocarboxylic acid (1b) is not particularly limited, it is preferably in a range of 90:10 to 10:90, more preferably in a range of 80:20 to 20:80.

The aliphatic monocarboxylic acid (1a) can be caprylic acid (15 to 17°C), capric acid (31°C), lauric acid (44 to 46°C), palmitoleic acid (5°C), oleic acid (16.3°C), linoleic acid (-5°C), linolenic acid (-11°C), and the like. These acids can be used singly or in combinations of two or more. It is to be noted that the temperature in parentheses is the melting point of the aliphatic monocarboxylic acid.

The aliphatic monocarboxylic acid (1b) can be myristic acid (54.5°C), palmitic acid (62.9°C), stearic acid (69.6°C), behenic acid (72 to 80°C), hydrogenated castor oil fatty acid, and the like. These acids can be used singly or in combinations of two or more. It is to be noted that the temperature in parentheses is the melting point of the aliphatic monocarboxylic acid.

In the present invention, various modifying agents, for example, waxes such as rice wax, carnauba wax, and dense wax, ethyl cellulose, propyl cellulose, polyethylene, chitosan and a derivative thereof, various polymers such as a pH sensitive polymer, a powder of an organic or inorganic material, a stabilizer, and a fragrance can be added as needed to modify moldability, mechanical strength, and other properties. Further, it is also possible to coat the surface of the calcium salt of an aliphatic monocarboxylic acid with these modifying agents.

The composition is preferably granular. Although the shape of the composition is not particularly limited, a shape with fewer edges such as a sphere, a spheroid, an artillery shell shape, and a cylinder is preferable. The size of the granular composition is not particularly limited as long as it is suitable as a feed component, and can be arbitrarily set in the range of the size of so-called granules to pellets. The granular composition preferably has such a size that specifically the particle diameter or length is 0.5 mm to 10 mm.

The method for producing the composition of the present invention is not particularly limited. Examples of the production method include granulation by extrusion. Specifically, a method involving kneading a biologically active substance and a protective substance, and then extruding the resulting kneaded product through a die having a certain size, followed by cutting with an air-cooled cutter can be employed. The kneading temperature is preferably 100 to 150°C, more preferably 110 to 135°C. The blower air temperature of the air-cooled cutter is preferably -10 to 40°C, more preferably 0 to 30°C, even more preferably 5 to 25°C. According to the method of the present invention, granular pieces cut with an air-cooled cutter do not stick together into a mass. In order to obtain a formulation with a small void fraction and less moisture content, it is preferable to carry out vacuum deaeration in the kneading step and rapidly cool the granular pieces by cold wind immediately after cutting with an air-cooled cutter. The granular composition produced as above can be directly used as a rumen-bypassing formulation. Also, this granular composition can be used as a core formulation of a coated formulation.

The formulation of the present invention contains the composition of the present invention. According to one embodiment, the formulation of the present invention may be the composition of the present invention formed into a granular shape. According to another embodiment, the formulation of the present invention is a coated formulation comprising a core formulation consisting of the composition of the present invention and a coating layer coating the core formulation, wherein the coating layer consists of at least one selected from the group consisting of a linear or branched, saturated or unsaturated aliphatic C₁₂₋₂₄ monocarboxylate, a polymer insoluble in a neutral region but soluble in an acidic region, and zein (hereinbelow, sometimes referred to as a coating agent).

Examples of the aliphatic monocarboxylate used in the coating layer include a calcium salt, a magnesium salt, an aluminum salt, and a zinc salt. Among them, a calcium salt is preferable. Examples of the calcium salt include the same substances as those exemplified as the calcium salt of an aliphatic monocarboxylic acid used as the protective substance above. While the aliphatic monocarboxylate used in the coating layer may be the same kind as or a different kind from the aliphatic monocarboxylate used as the protective substance in the core formulation, the same kind is preferable.

Also, examples of the polymer insoluble in a neutral region but soluble in an acidic region and zein include a cellulose derivative such as benzylaminomethyl cellulose, dimethylaminomethyl cellulose, piperidylethylhydroxyethyl cellulose, cellulose acetate diethyl aminoacetate, and cellulose acetate dibutylaminohydroxypropyl ether, a polyvinyl derivative such as a vinyl diethylamine-vinyl acetate copolymer, a vinyl benzylamine-vinyl acetate copolymer, polyvinyldiethylaminoacetoacetal, a vinylpiperidylacetoacetal-vinyl acetate copolymer, polyvinylacetal diethylaminoacetate, polydimethylaminoethylmethacrylate, polydiethylaminomethylstyrene, polyvinylethylpyridine, a vinylethylpyridine-styrene copolymer, a vinylethylpyridine-acrylonitrile copolymer, a methylvinylpyridine-acrylonitrile copolymer, and a methylvinylpyridine-styrene copolymer, chitosan, a metal salt of polysaccharides such as calcium alginate, and a water-insoluble metal salt of an acid which is less acidic than hydrochloric acid and is acceptable to the living body such as calcium carbonate, tricalcium phosphate, dicalcium phosphate, trimagnesium phosphate, zinc phosphate, aluminum phosphate, calcium silicate, calcium pyrophosphate, magnesium carbonate, lead carbonate, and cobalt carbonate.

The coating layer can be formed by, for example, mixing and then stirring, while heating, a core formulation consisting of the composition of the present invention and a powder of the aforementioned coating agent.

The particle size of the powder of the coating agent is preferably such a size that it passes through a 50-mesh sieve, more preferably such a size that it passes through a 100-mesh sieve.

Stirring can be performed by a method involving operations such as putting the mixture in a container and rotating the container.

The heating temperature is preferably set higher than the softening temperature (melting point) of the core formulation (aliphatic monocarboxylic acid) and lower than the softening temperature (melting point) of the coating agent. When the heating temperature is too low, it becomes difficult to achieve sufficient attachment of the powder of the coating agent. When the heating temperature is too high, the core formulations, the coating agent powders, or the formulations aggregate together and attach to the heating container, easily causing obstacles in the coating operation.

The amount of the coating agent is preferably 2 parts by mass or more, more preferably 2 to 20 parts by mass, even more preferably 3 to 15 parts by mass based on 100 parts by mass of the core formulation. Since there is an upper limit to the amount of the coating agent coating the core formulation, the formulation may contain a coating agent which is not involved in coating of the core formulation. However, even if a coating agent not involved in coating is contained in the formulation of the present invention, the effect of the present invention is not affected.

The formulation of the present invention has a low elution rate in the rumen, and thus is suitable for rumen bypass.

### Examples

The present invention will be described further in detail with illustrative Examples. However, the scope of the present invention is not limited in any way by the following Examples. In the present Examples, a "part" means "part by mass," unless otherwise specifically noted.

In the present Examples, the following measurements were carried out.

### (Calcium content of a fatty acid salt)

1 g of a fatty acid salt was incinerated at 550°C. The resulting incinerated product was dissolved in hydrochloric acid, and then diluted to a predetermined dilution factor with water. The resulting diluted solution was quantitatively analyzed by an ICP emission spectrometer to calculate the calcium content based on the dry weight of the fatty acid salt.

### (Moldability into granules)

The state in which the granular pieces cut with an air-cooled cutter did not stick together into a mass was graded "good", while the state in which the granular pieces cut with an air-cooled cutter stuck together or formed a mass was graded "poor".

### (Elution rate in the rumen (PB value))

Formulations were immersed in a pseudo-rumen fluid and shaken for 16 hours. Subsequently, the mass of the active substance eluted was measured. The ratio of the mass of the active substance eluted to the mass of the active substance in the formulation was calculated and the value thus obtained was determined as the elution rate in the rumen (PB value). The smaller the PB value, the better the rumen-bypassing efficiency.

It is to be noted that, as a pseudo-rumen fluid, a solution obtained by dissolving 9.3 g of disodium hydrogen phosphate dodecahydrate and 9.8 g of sodium bicarbonate in water and bringing the total volume to 1 L was used. The pH of this solution was 6.4.

### Example 1

20 parts of a calcium salt of a palm oil fatty acid (purity: 94.0%, calcium content: 9.8%), 6 parts of lauric acid, 2 parts of stearic acid, and 72 parts of methionine (bulk specific gravity: 0.684, containing 60.9% by mass of particles having a diameter of 250 µm or greater) were mixed. The resulting mixture was placed in the hopper of a biaxial extrusion granulator and kneaded at 120°C. The resulting kneaded product was melt-extruded through a die having a hole diameter of 1.6 mm while performing vacuum deaeration, and then cut with an air-cooled cutter with a blower air temperature of 22°C to obtain artillery shell-shaped core formulations having an average major axis of 1.6 mm and an average length of 1.6 mm.

SEM observation of the cross-section of the formulations revealed that methionine was dispersed in a matrix consisting of a calcium salt of a palm oil fatty acid, lauric acid, and stearic acid.

The calcium salt of a palm oil fatty acid was pulverized to obtain a powder [A] passing through a 100-mesh sieve. Into a 1 L flask, 20 parts of the aforementioned core formulations and 2 parts of the powder [A] were placed and the contents were heated to 80°C while rotating the flask, whereby the powder [A] was attached to the core formulations to form a coating layer.

### Examples 2 to 12

Formulations were obtained by the same procedure as in Example 1, except for changing the prescriptionss to the ones shown in Tables 1 and 2. The PB values of the resulting formulations are shown in Tables 1 and 2. As in Example 1, SEM observation of the cross-section of the formulations revealed that methionine was dispersed in a matrix consisting of a calcium salt of a palm oil fatty acid and the like.

### Comparative Examples 1 and 2

An attempt was made to obtain formulations by the same procedure as in Example 1, except for changing the prescriptions to the ones shown in Table 2. However, the products could not be cut with an air-cooled cutter, resulting in a failure to obtain granules.

[Table 1]

**Table 1**

| | Example | | | | | | |
|---|---|---|---|---|---|---|---|
| [Part by mass] | 1 | 2 | 3 | 4 | 5 | 6 | 7 |
| Calcium salt of a palm oil fatty acid with a Ca content of 9.8% | 20 | 20 | - | - | - | 21 | - |
| Calcium salt of a palm oil fatty acid with a Ca content of 8.5% | - | - | 20 | 24 | 22.7 | - | 14 |
| Methionine | 72 | 72 | 72 | 72 | 72 | 72.3 | 78 |
| Lauric acid | 6 | 4 | 2 | 3 | 4 | 5 | 6 |
| Stearic acid | 2 | 4 | 6 | 1 | 1.3 | 1.7 | 2 |
| Moldability into granules | Good | Good | Good | Good | Good | Good | Good |
| PB value | 10 | 15 | 13 | 18 | 17 | 18 | 8 |

[Table 2]

**Table 2**

| | Example | | | | | Comparative Example | |
|---|---|---|---|---|---|---|---|
| [Part by mass] | 8 | 9 | 10 | 11 | 12 | 1 | 2 |
| Calcium salt of a palm oil fatty acid with a Ca content of 9.8% | - | - | - | 22.5 | - | - | - |
| Calcium salt of a palm oil fatty acid with a Ca content of 8.5% | 18.6 | 20 | 20 | - | 23 | 20 | - |
| Calcium salt of a palm oil fatty acid with a Ca content of 7.5% | - | - | - | - | - | - | 20 |
| Methionine | 74 | 72 | 72 | 70 | 70 | 72 | 74 |
| Lauric acid | 5.6 | 6 | 6 | 5.625 | 5.25 | 8 | 6 |
| Stearic acid | 1.8 | - | - | 1.875 | 1.75 | - | - |
| Palmitic acid | - | 2 | - | - | - | - | - |
| Myristic acid | - | - | 2 | - | - | - | - |
| Moldability into granules | Good | Good | Good | Good | Good | Poor | Poor |
| PB value | 12 | 12 | 7 | 17 | 11 | - | - |

From the results shown above, it was found that by using a combination of an aliphatic monocarboxylic acid having a melting point of lower than 50°C and an aliphatic monocarboxylic acid having a melting point of 50°C or higher, uniformly shaped granules were successfully obtained even when cutting was performed with an air-cooled cutter, and moreover, that the resulting granules had favorable rumen-bypassing efficiency.

## Claims

**1.** A composition comprising:
10 to 50% by mass of a protective substance comprising a linear or branched, saturated or unsaturated aliphatic C₈₋₂₄ monocarboxylic acid (1a) having a melting point of lower than 50°C, a linear or branched, saturated or unsaturated aliphatic C₈₋₂₄ monocarboxylic acid (1b) having a melting point of 50°C or higher, and a calcium salt of a linear or branched, saturated or unsaturated aliphatic C₁₂₋₂₄ monocarboxylic acid (2) having a calcium content of 8 to 12% by mass, and
50 to 90% by mass of a biologically active substance dispersed in the protective substance.

**2.** The composition according to claim 1, wherein a ratio of a total mass of the aliphatic monocarboxylic acid (1a) and the aliphatic monocarboxylic acid (1b) with respect to a mass of the calcium salt of an aliphatic monocarboxylic acid (2) is 40/60 to 10/90.3. The composition according to claim 1 or 2, wherein a ratio of a mass of the aliphatic monocarboxylic acid (1a) with respect to a mass of the aliphatic monocarboxylic acid (1b) is 80/20 to 20/80.

**4.** The composition according to any one of claims 1 to 3, wherein the biologically active substance is an amino acid.

**5.** The composition according to any one of claims 1 to 3, wherein the biologically active substance is at least one selected from the group consisting of methionine and lysine hydrochloride.

**6.** The composition according to any one of claims 1 to 5, wherein the aliphatic monocarboxylic acid (1a) is lauric acid.7. The composition according to any one of claims 1 to 6, wherein the aliphatic monocarboxylic acid (1b) is at least one selected from the group consisting of palmitic acid, myristic acid, and stearic acid.

**8.** The composition according to any one of claims 1 to 7, wherein the calcium salt of an aliphatic monocarboxylic acid (2) is a calcium salt of a palm oil fatty acid.

**9.** A method for producing a composition according to any one of claims 1 to 8, comprising the steps of:
kneading and extruding, by an extrusion kneading machine,
50 to 90% by mass of a biologically active substance and
10 to 50% by mass of a protective substance comprising a linear or branched, saturated or unsaturated aliphatic C₈₋₂₄ monocarboxylic acid having a melting point of lower than 50°C, a linear or branched, saturated or unsaturated aliphatic C₈₋₂₄ monocarboxylic acid having a melting point of 50°C or higher, and a calcium salt of a linear or branched, saturated or unsaturated aliphatic C₁₂₋₂₄ monocarboxylic acid having a calcium content of 8 to 12% by mass; and
cutting a resulting product with an air-cooled cutter.

**10.** A formulation consisting of a composition according to any one of claims 1 to 8.

**11.** A coated formulation comprising:
a core formulation consisting of a composition according to any one of claims 1 to 8 and
a coating layer coating the core formulation, wherein the coating layer consists of at least one selected from the group consisting of a linear or branched, saturated or unsaturated aliphatic C₁₂₋₂₄ monocarboxylate, a polymer insoluble in a neutral region but soluble in an acidic region, and zein.

**12.** The coated formulation according to claim 11, wherein an amount of the coating layer is 2 to 20 parts by mass based on 100 parts by mass of the core formulation.

**13.** The formulation according to any one of claims 10 to 12, wherein the formulation is for rumen bypass.
